# EUROPEAN PATENT APPLICATION

(11) **EP 4 632 064 A1**
(43) Date of publication of application: **15.10.2025**
(21) Application number: 25164902.6
(22) Date of filing: 20.03.2025
(51) Int. Cl.: C12N 5/0783, C12N 5/09, C12N 5/071

(54) **A PATIENT-DERIVED EX-VIVO TUMOR TISSUE MODEL**

(30) Priority: 21.03.2024 EP 24165260
(71) Applicant: Wesch, Daniela, 24105 Kiel (DE); Oberg, Hans-Heinrich, 24105 Kiel (DE); Bauerschlag, Dirk, 24105 Kiel (DE)
(72) Inventor: Wesch, Daniela, 24105 Kiel (DE); Oberg, Hans-Heinrich, 24105 Kiel (DE); Bauerschlag, Dirk, 24105 Kiel (DE)
(74) Representative: Moré, Solveig Helga

(57) **Abstract**

The present invention relates to the field of personalized medicine in oncology, in particular, to a patient-derived ex-vivo tumor tissue model provided. The invention provides a culture of patient-derived tumor cells comprising complete dissociated tumor tissue, and a method of using the same for determining the effect of a potential therapeutic agent on cytotoxicity of viable, resting, untouched tumor-infiltrating lymphocytes (TIL) or other viable, resting, untouched tumor-infiltrating immune cells of a patient having a solid tumor and/or for selecting a therapeutic agent effective in a particular patient. The invention also provides a method of treating a patient having a solid tumor with the selected therapeutic agent. Screening of potential therapeutic agents and/or optimized and personalized treatment of a patient are rendered possible in an easy test that can be carried out, e.g., in the course of one to two weeks.

## Description

The present invention relates to personalized medicine in the field of oncology, in particular, to a patient-derived ex-vivo tumor tissue model (exTuTiMo). The invention provides a culture of patient-derived tumor cells comprising complete dissociated tumor tissue, and a method of using the same for determining the effect of a potential therapeutic agent on cytotoxicity of tumor-infiltrating lymphocytes (TIL) or other tumor-infiltrating immune cells of a patient having a solid tumor and/or for selecting a therapeutic agent effective in a particular patient. The invention also provides a method of treating a patient having a solid tumor with the selected therapeutic agent. Screening of potential therapeutic agents and/or optimized and personalized treatment of a patient are rendered possible in an easy test that can be carried out, e.g., in about one to two weeks.

The standard cancer therapy is often surgical resection followed by chemotherapy and/or irradiation or neoadjuvant therapy followed by surgery. A great problem for an effective treatment is the recurrence of disease even after successful surgical treatment. This relapse is often caused by a resistance against current chemotherapeutic standard regimen. Therefore, alternative treatment options based on activation of the immune system are emerging, e.g., therapy with checkpoint inhibitors, new design of T Cell Engagers (TCE) such bispecific antibodies (bsAb) targeting different tumor-associated antigens alone or in combination with chemotherapy, CAR T cell therapy or allogeneic γδ T cell adoptive cell therapy. Cancer immunotherapy attempts to stimulate the immune system to destroy tumors. A variety of strategies are in use or are undergoing research and testing. Randomized controlled studies in different cancers resulting in significant increase in survival and disease-free period have been reported, and its efficacy is enhanced by 20-30% when cell-based immunotherapy is combined with conventional treatment methods (Syn et al., 2017. The Lancet. Oncology 18(12):e731-e741, doi:10.1016/s1470-2045(17)30607-1).

The use of monoclonal antibodies (mAb) such as rituximab in cancer therapy was one of the first introduced in 1997. Rituximab is a humanized anti-CD20 mAb for treatment of B cell lymphoma and leukaemia. Since then, several monoclonal and bispecific antibodies have been approved for treatment of various haematological malignancies. For instance, blinatumomab with a specificity for CD3 on T cells and for CD19 on lymphoma or leukaemia cells have proved to be efficient for the treatment of patients with haematological malignancies. In contrast to haematological malignancies, the treatment of solid tumors by T Cell Engagers is delayed. Klein et al. (Nature Reviews Drug Discovery, 2024, https://doi.org/10.1038/s41573-024-00896-6) summarized that 14 bsAb have been approved (11 for treatment of cancer) and others are in clinical development.

However, efficacy of cancer treatment varies greatly from patient to patient. This also applies for immunological cancer treatments. One reason for this is seen in different immunomodulatory characteristics of individual tumors. There is thus a need in the art for testing potential therapeutic agents for treatment of cancer, also taking the characteristics of individual tumors into account, e.g., to find out which patients would benefit from a specific treatment.

Different models for testing efficacy of agents on patient-derived tumor cells have been developed. Talwelkar et al. isolated EpCAM-positive tumor cells and EpCAM-negative stroma cells from freshly dissociated tumor material to compare the effect of medicaments on these both populations (Talwelkar et al., 2021, Cell Rep Med 2:100373. doi: 10.1016/j.xcrm.2021.100373; Talwelkar et al, 2022. STAR Protoc 3:101720. doi: 10.1016/j.xpro.2022.101720.

Duan et al., 2020. (Trends Cancer 6, :605-618, doi: 10.1016/j.trecan.2020.02.022) show that the biggest challenge in the efficacy of cancer therapy is the heterogeneity of the immunosuppressive tumor microenvironment, which can reduce the number of tumor-infiltrating immune cells or lead to their exhaustion. Different mechanisms of immunosuppression are known, e.g., in suppression of γδ T-cell activity (Wesch et al., 2020. Immunological Reviews. 298:84-98. dOl: 10.1111/imr.12925). Gene-editing of tumor infiltrating lymphocytes and use of such gene-edited cells in immunotherapy has been suggested (WO 2021/081378 A1)

Cytotoxic capacity of lymphocytes from blood or TIL, or short-term cultures thereof, in the presence or absence of a therapeutic agent, can be determined in vitro in cytotoxicity assays using patient-derived tumor cells or tumor cell lines as targets in a defined effector-target ratio (Oberg etal., 2014, Cancer Res 74:1349-60, doi: 10.1158/0008-5472.CAN-13-0675; Oberg et al., 2016, Pancreatology16:1069-1079, doi:10.1016/j.pan.2016.07.008. ; Oberg et al., 2019, J Leukoc Biol 107:1081-95, doi: 10.1002/JLB.5MA1119-265R). Saleh et al. (2020. Genes 11:703. doi: 10.3390/genes11060703) use dissociated tumor material having an immunosuppressive tumor microenvironment for transcription analysis.US 2023258624 A1 teaches an ex vivo tumour immune microenvironmental model comprising patient-derived explants in a cellulose hydrogel matrix.

In an attempt to provide a model of the tumor microenvironment, xenografts of patient-derived tumor xenografts have been tested in partly or fully humanized mouse models, e.g., by Ito et al., 2002, Blood 100:3175-3182, doi: 10.1182/blood-2001-12-0207; Morton et al., 2016. Cancer Res 76:6153-6158, doi: 10.1158/0008-5472; Audigé et al, 2017. BMC Immunol, 18:28, doi: 10.1186/s12865-017-0209-9; Odunsi et al., 2020. Journal for Immunotherapy of Cancer; 8: e001237, doi:10.1136/jitc-2020-001237; Lin et al, 2020. CANCER BIOLOGY & THERAPY 21:130-138, doi.org/10.1080/15384047. 2019.1670520). Such models need a high number of mice, have a low tumor diversity and several weeks are required to get results, so this is very expensive. Further, mouse models, even if humanized, do not fully represent the human system and the complete tumor microenvironment, and results between such models and clinical studies in humans often differ (Horvath et al., 2016, Nat Rev Drug Discov 15:751-769; Bibby et al., 2004. Eur. J. Cancer 40:852-857, Ho & Msallam, 2021, Cells 10:831-849, doi.org/10.3390/ cells10040831).

In vitro 3D models using human organoids or spheroids require several weeks for growth of tumor cells. TIL often die during the co-culture. Therefore, TIL often cannot be used shorty after their ex vivo isolation. They have to be frozen up for the use of co-cultivation with organoid tumors (Neal et al., 2018, Cell 175:1972-1977, doi:10.1016/ j.cell.2018.11.021; Ho & Msallam, 2021, Cells 10:831-849. doi.org/10.3390/cells10040831). WO 2021/081491 A1 describes patient-matched organoid systems for studying cancer. WO 2021/092555 A2 teaches methods of screening to determine effective dosing of cancer therapeutics comprising formation of a cancer spheroid. Hong Nam Kim et al., 2019 (Advances functional materials, Wiley - VCH Verlag GmbH & Co KGAA, DE, Vol. 29, No. 22) considers 3D microphysiological systems as enabling tools for modelling complexity in the tumor microenvironment and accelerating cancer drug development. Like the mouse models, 3D organoid models provide good systems for testing different hypothesis, as they partially mirror characteristics of the tumor microenvironment in a controlled system where, e.g., certain parameters can be varied to determine their effect. However, these models also do not always represent a realistic immunosuppressive tumor microenvironment, and they can be very time-intensive to generate.

In light of the state of the art, the inventors addressed the need to provide a model system that overcomes at least some of these drawbacks, in particular, a model system that allows for assessing the effect of a potential therapeutic agent on cytotoxic capacity of patient-derived tumor-infiltrating lymphocytes, taking the immunosuppressive environment of the tumor into account and providing results within a shorter time frame, with lower expense and effort and/or by simpler means compared to previous model systems.

This problem is solved by the present invention, in particular, by the subject-matter of the claims. The invention provides a method for determining the effect of a potential therapeutic agent on cytotoxicity of TIL or other tumor-infiltrating immune cells of a human patient, having a solid tumor, comprising steps of
a) dissociating a sample derived from the tumor of the patient to produce cells of the tumor sample;
b) separating dead cells and debris to purify complete dissociated tumor tissue;
c) cultivating, in absence of a potential therapeutic agent or in presence of at least one potential therapeutic agent, the complete dissociated tumor tissue without changing the ratio between different subsets of living cells, thereby producing a complete dissociated tumor tissue culture; and
d) determining lysis and/or proliferation of cells in the complete dissociated tumor tissue culture, wherein the lysis of tumor cells is a measure of the cytotoxic effect of the TIL or the other immune cells.

The method of the invention is of highest relevance in the human system, as it provides a model system that can, within a short time span such as about a week, and with straight-forward, comparatively cost-effective and easy to establish methods, assess the effect of a potential therapeutic agent on the tumor cells, TIL, other immune cells, such as associated leukocytes (e.g. myeloid derived suppressor cells, NK cells, Mucosal-associated invariant T cells (MAIT cells) or a combination of such cells) of a patient. Advantageously, this is in the presence of the stromal and epithelial compartment (i.e., the tumor-microenvironment, TME). Typically, the cytotoxicity of tumor-infiltrating lymphocytes (TIL) is determined, and the lysis of proliferation of tumor cells is a measure of the cytotoxic effect of the TIL. While not explicitly mentioned in the following, other tumor-infiltrating immune cells, e.g., macrophages, Natural Killer cells; MAIT cells or rare T cells forming a link between innate and adaptive immune cells such as γδ T cells, e.g., γδ TIL, can also be activated by appropriate therapeutic agents to cause cytotoxicity in the assay of the invention, and this can be assessed by the same means as with TIL.

In contrast to assays known in the art, the tumor-infiltrating lymphocytes (TIL) or other tumor-infiltrating immune cells used for step c) are viable.

They are also resting (i.e., freshly isolated and not expanded in culture). Resting also implies that they have not been stimulated by acting any agent before addition of the potential therapeutic agent for which the effect is to be determined. Preferably, the tumor-infiltrating lymphocytes (TIL) or other tumor-infiltrating immune cells used for step c) are further untouched (i.e., not embedded in a matrix gel). Advantageously, these cells (and indeed all cells of the complete dissociated tumor tissue), are viable, resting and untouched.

Therefore, the use of different Immune Cell Engagers together with freshly isolated tumor tissue and untouched cells in the inventive assay also provides information about which are the most potent immune cells. Interestingly, so far, the inventors found that in a donor the responder cells can sometimes be NK cells or αβ- or γδ T cells or MAIT cells or a combination of some of these cells. The assay can figure out within a short time span, e.g., a week or 14 days, which are the best responder immune cell population within the TIL surrounded by tumor cells and TME. The invention thus provides use of the method of the invention for determining the best responder immune cell population within the TIL of a patient.

The presented assay allows to determine whether a potential therapeutic agent, e.g., an Immune Cell Engager (i.e., an agent selectively targeting immune cells to tumor cells, e.g., a bispecific binding agent), such as a T cell engager, activates the expansion of regulatory T cells within a short time span, e.g., a week or 14 days. These data are very important for a successful treatment, since regulatory T cells are described to inhibit anti-tumor responses. The invention thus provides use of the method of the invention for determining if a potential therapeutic agent activates regulatory T cells.

The effect on the TIL is assessed by testing the cytotoxic function of these cells, i.e., by determining lysis and/or proliferation of tumor cells in the culture. Typically, the tumor cells in the culture, which can adhere to the bottom of the cell culture vessel, are lysed by the TIL if these have cytotoxic function. Thus, the assay demonstrates the effect of the potential therapeutic agent on cytotoxicity of the TIL. The measurement of the functional capacity (e.g., cytotoxicity) of untouched TIL, in particular, TIL that are not embedded in a matrix gel and not cultured over weeks, is a crucial advantage of the inventive method. This has not been determined in any other assay so far.

Further, advantageously, the effect, in particular, the effect on cytotoxicity, can be determined at different time points or even over a time span, e.g., over the time the assay is carried out, e.g., for at least a day, or 1-14 days, e.g., 5-10 or 5-7 days or about a week. Thus, the longitudinal effect can be determined, not just an effect at one specific time point.

Other parameters, e.g., phenotypic or other functional characteristics of the TIL can also be determined. The assay can further assess, e.g., if tumor cells are able to regenerate and/or proliferate, e.g., if the TIL do not have a strong enough cytotoxic effect or get exhausted under the tested conditions. The method of the invention thus allows for selection of an effective treatment of said patient. The time required for this method is comparatively short and thus optimizes the timing of patient treatment with the individually selected, optimized treatment agent. The method can also be used for assessing the effect of a potential therapeutic agent in a number of patient-derived tumor samples and thus help to determine if the therapeutic agent is effective in a sufficiently high proportion of tumors to warrant further investigation and/or clinical use.

Further, compared to xenograft mouse models, the model system of the invention represents a completely human and autologous model. These is no graft-versus host reaction possible, and an influence of murine cells is excluded. In comparison to 3D models, the present invention can, directly after obtaining and dissociating the tumor material, determine cytotoxic activity of TIL with the tumor cells as target cells, taking the potential negative effects of the immunosuppressive tumor microenvironment into account in a completely autologous system. The possibility of mirroring the complete immunosuppressive tumor microenvironment is an essential feature of the inventive method, and the method thus provides individual results on the effect of the tumor microenvironment on the capacity of TIL. As the ratio of cell subsets, in particular, of effector cells, i.e., cytotoxic immune cells such as TIL and of target cells (tumor cells) is not changed in the assay compared to the ratio in the tumor, the situation in the tumor tissue is optimally mirrored and the method provides a more realistic prediction on the effects of the potential therapeutic agent.

Solid tumors, in the context of the invention, are malignant tumors, i.e., cancers. The tumor can be a carcinoma, e.g., adenocarcinoma, a sarcoma, a germ cell tumor or a blastoma, e.g., lung cancer, colon cancer, colorectal cancer, pancreatic cancer, prostate cancer, gastric cancer, breast cancer, cervical cancer, ovarian cancer, squamous cell carcinoma of the head and neck, hepatocellular carcinoma, melanoma, or glioma.

TIL are lymphocytes that infiltrate the tumor, i.e., lymphocytes that can be purified from a solid tumor. In the context of the present invention, however, no purification from other cells of the tumor tissue is required. They include T cells and B cells and are part of the larger category of 'tumor-infiltrating immune cells' which consist of both mononuclear and polymorphonuclear immune cells, (i.e., T cells, B cells, natural killer cells, macrophages, neutrophils, dendritic cells, mast cells, eosinophils, basophils, etc.) in variable proportions. Their abundance varies with tumor type and stage and in some cases relates to disease prognosis.

TIL can often be found in the tumor stroma and within the tumor itself. TIL are implicated in killing tumor cells. The presence of lymphocytes in tumors is often associated with better clinical outcomes. Their functions can dynamically change throughout tumor progression and in response to anticancer therapy. It is thus important to test the functionality of the TIL of a patient, in particular, their capability of responding to tumor antigens in the immunosuppressive microenvironment of the tumor. This capability can sometimes be improved by therapeutic agents, but it has been found that the effectiveness of therapeutic agents strongly differs between patients. The cytotoxic capability of TIL of a patient, and an effect of potential therapeutic agent on this capability can advantageously be tested using the method of the present invention, as in the method of the invention, all cells of the tumor environment are present and can, as in the in situ situation, affect the function of the TIL.

The potential therapeutic agent can be any agent suspected to have an effect on the survival of tumor cells, in particular, an agent suspected to improve at least one aspect of an immune response detrimental to tumor cells, e.g., an immunotherapeutic agent. A combination of potential therapeutic agents can also advantageously be tested by the method of the invention. As a control, a complete dissociated tumor tissue culture in the absence of the potential therapeutic agent (and, preferably, if several agents are combined, also with separate agents) should also be cultivated and assessed.

There are several types of immunotherapeutic agents that can be used to treat cancer and tested in the assay of the invention:
- Immune cell engagers, (ICE, i.e., an agent selectively targeting immune cells to target cells), preferably, T cell engagers (also designated TCE), are multispecific (e.g., bispecific or trispecific) antibody constructs that comprise at least 2 antigen-binding sites, wherein at least one antigen binding site is capable of specifically binding an antigen expressed on the surface of tumor cells, preferably, a tumor-specific antigen, and wherein at least one other antigen-binding site is capable of specifically binding an immune cell, preferably, a T cell activating molecule. The antibody construct can comprise, e.g., scFv, single domain antibodies or Fab fragments having the recited respective binding sites. A bispecific T cell engager can also be designated bsTCE or BiTE.
   The antigen expressed on the surface of tumor cells can be, e.g., HER2, EGFR or EpCAM, if the tumor is an ovarian cancer, HER2, EGFR, CEA and EpCAM in breast cancer, CEACAM5 if the tumors are a lung-, gastric- or colorectal cancer. Thus, for analysis of T cells in ovarian, breast or pancreatic cancer, e.g., a bispecific T cell engager designated bsTCE [HER2×CD3] can be used, wherein one binding site is capable of binding to HER2 and one binding site is capable of binding to CD3. Consequently, after binding of the T cell engager to both antigens, the T cell is activated via CD3. T cell activation with T cell engagers is thus typically independent of the antigen specificity of the TCR. Another bsTCE [(HER2)₂×Vγ9] only engages γδ T cells by binding to Vy9 T Cell Receptor (TCR) and with two binding sites to HER2. bsTCE which can be used in the context of the invention are disclosed in more detail, e.g., in Oberg et al., 2014. Cancer Res; 74(5); 1-12. DOI:10.1158/0008-5472.CAN-13-0675; Oberg et al., 2019. J Leukoc Biol. 2019;1-15. DOI: 10.1002/JLB.5MA1119-265R.
   Bispecific antibodies are available in different formats, target different tumor-(associated) antigens and enhanced cytotoxicity of immune cells.
- Immune system modulators: agents such as cytotoxic or cytostatic agents, tyrosine kinase inhibitors, immune check point inhibitors or Toll-like receptors ligands alone or in combination with ICE, preferably, TCE that enhance the body's immune response against cancer. Immune checkpoint inhibitors are preferred immune system modulators: Immune checkpoint inhibitors are drugs that block immune system checkpoints to allow immune cells to respond more strongly to the cancer. Any checkpoint inhibitors known in the art can be used, e.g., α-PD-1 (programmed cell death protein 1), α-PD-1L (programmed cell death protein 1 ligand), α-TIM-3 )T-cell immunoglobulin and mucin-domain containing-3, also designated hepatitis A virus cellular receptor 2), α-CTLA-4 (cytotoxic T-lymphocyte associated protein 4), α-LAG-3 (lymphocyte-activation gene 3), α-TIGIT (T cell immunoreceptor with Ig and ITIM domains)), α-BTLA (B- and T-lymphocyte attenuator, also designated CD272) or α-VISTA (V-domain Ig suppressor of T cell activation).
- In contrast, monotherapy with conventional IgG-based monoclonal antibodies designed to bind to specific targets on cancer cells. The targets can be antigens expressed on the surface of tumor cells. Exemplary antibodies binding to HER2 are trastuzumab and pertuzumab which are applied against metastatic breast and gastric cancer. Trastuzumab and pertuzumab induce cell death or Ab-dependent killing as well as novel trastuzumab emantansine which stops the HER2 signal and destroy tubulin of tumor cells.
- T cell transfer therapy: a treatment that takes T cells or subpopulations thereof, e.g., from the tumor and selects, expands and/or genetically engineers them ex vivo to better attack cancer cells, then reintroduces them into the patient. T cells can also be derived from blood, or even be heterologous T cells (e.g., gamma delta T cells). T cells can be engineered to express, e.g., a CAR or a TCR capable of targeting a tumor antigen or an epitope from a tumor antigen in association with an MHC molecule expressed by the tumor cells.

Different therapeutic agents, e.g., different ICE, preferably, TCE, can be used to selectively activate different immune cell subpopulations and to determine their cytotoxic capacity under the conditions of the immunosuppressive microenvironment. For example, ICE or TCE can specifically target all T cells, γδ T cells, αβ T cells, NK cells or NKT and MAIT cells or T cells expressing specific co-stimulatory molecules.

After a tumor sample is derived from the patient, the cells of the sample are dissociated. The tumor sample can be derived from a biopsy or from surgery largely or essentially removing a solid tumor. The tumor can be metastatic or non-metastatic. Early stage tumor samples can be assessed using the method of the invention. The cancer can also be in an advanced stage if the method of the present invention is carried out to determine personalized treatment options.

The tumor sample should be fresh. Preferably, the culture of the complete dissociated tumor tissue is started the same day the tumor sample has been isolated from the patient, i.e., the cells are seeded into the culture plate the same day.

The dissociation has to be carried out on the same day as the dissection of the tumor sample. The sample should be cooled, e.g., kept on ice or at 0-8°C. Freezing the dissociated tumor sample is possible, but freezing or prolonged storage may compromise the vitality quality of cells.

The dissociation of the tumor sample comprises mincing and enzymatically dissociating the sample. Before the enzymatic treatment, the tumor and/or tumor pieces are washed, e.g., with PBS, to remove remains of blood. While commercial kits, e.g., as described in the examples below, can be used for dissociating the tumor samples, this can also be performed using enzymes know in the state of the art for this purpose. For example, the inventors have also received good results dissociating the tumor samples with a combination of collagenase and DNAse. Optionally, the obtained cells are further passed through a mesh, e.g., 100 µm mesh. This already removes large cell clumps and debris.

The inventors have found the reliability of the assay, the vitality of the cells during the assay and the reproducibility improved if dead cells and debris are separated by density gradient separation, e.g., Pancoll^{®}. The density gradient may comprise a medium, e.g., a sugar solution having a density of 1.077 g/mL and a medium having a density of about 1 g/mL. Dead cells and debris are then found in the pellet, while tumor cells, TIL and tumor microenvironment (TME) are found in the intermediate layer. The inventors have tested by phenotypic analyses that the dead cells that are separated in this step are largely tumor cells, which - without intending to be held by the theory - are believed to die after dissection of the tumor sample.

The cell composition generated after dissociation and separation of dead cells and debris is designated complete dissociated tumor tissue. It comprises all living cells from the tumor in the composition also contained in the tumor, i.e., without changing the ratio, e.g., of TIL to tumor cells. Thus, the microenvironment of the tumor is optimally represented. Further, the number of TIL is not artificially increased. In state of the art TIL cytotoxicity assays, the number of TIL is typically much higher [e.g., effector (TIL) to target (tumor cell) ratio 50:1 to 1:1] than in the cultures obtained according to the present invention, where the effector target ratio can e.g., vary between 10:1 to 1:90 for αβ T cells and 1:200 to 1:5000 for γδ T cells.

The inventors have further found out that the concentration in which the complete dissociated tumor tissue is seeded is important for the quality of results. This may depend on the readout method of the cytotoxicity test and the culture plates used, but, in particular if impedance measurements are used to determine lysis of tumor cells, a culture at 1*10⁵ to 4*10⁵ cells/well of a 96 well-plate is recommended, preferably, at 2 to 2.5*10⁵ cells/well, so the cells are seeded at this concentration.

In a preferred embodiment, the cells are seeded in an E-plate, i.e., a plate comprising, in each well, a sensor capable of measuring impedance, preferably, gold sensors.

While the lysis of the cells or cell death can be determined based on any suitable method, e.g., cell adhesion, membrane permeability of a dye or on release of an enzyme, a fluorescent marker or a radioactive marker from lysed cells, preferably, no labelling or marker is used. Preferably, throughout the invention, lysis of cells is determined based on cell adhesion. Additionally, viable (absolute) number of tumor cells can be determined before and after culturing by flow cytometry.

This determination based on cell adhesion advantageously employs non-invasive electrical impedance monitoring. Microtiter plates (e.g., E-plates) which contain gold biosensors embedded in the bottom of each well are used for this complete dissociated tumor tissue culture. These biosensors continuously and non-invasively monitor changes in cell number, cell size, cell barrier function, and cell-substrate attachment quality.

Within the wells of said microtiter plate, adherent cells act as insulators, restricting the flow of a miniscule electric current between the biosensors. This signal is measured automatically, at a frequency defined by the user (every 10 seconds, once per hour, etc.), and provides a dynamic view of cell health and behaviour at an unprecedented level of detail to quantify cell proliferation, morphology change, and attachment quality in a label-free, real-time manner. The instrument can be placed in a standard CO₂ cell culture incubator and may interface via a cable with analysis and control units that can be housed outside the incubator. Software allows for real-time control and monitoring of the instrument, and may include real-time data display and analysis functions.

In the present method, the inventors have found that the impedance is essentially affected by the presence of viable tumor cells which adhere to the bottom of the wells. When these tumor cells are lysed, e.g., by the TIL or by addition of detergent as a control, the insulating adherent cells are removed, so that lysis can conveniently be monitored over time. This enables an easy method to control for the effect of agents over time, taking, e.g., potential regrowth of tumor cells into account. Importantly, the inventive method describes for the first time the measurement of the cytotoxic capacity of untouched TIL in a potential untouched immunosuppressive TME. Thereby the assay allows a functional characterisation of these untouched TIL.

An advantageous system for electrical impedance monitoring of lysis of tumor cells is provided by using a Real-Time Cell Analyzer (RTCA) system, in particular, the W380 xCELLigence^{®} RTCA (Agilent/ OLS/ genomax). Thus, preferably, the lysis of adherent tumor cells is determined based on an impedance measurement, wherein, preferably, lysis of the tumor cells is determined over the course of the culture. The inventors analysed that monocytes within TIL or PBMC did not adhere appropriately to the plates and thus did not affect the measurement.

If lysis of tumor cells is determined by measuring impedance, a baseline value for impedance is optimally determined after adherence of the tumor cells to the plate. Preferably, the potential therapeutic agent is added to the culture after the tumor cells have adhered to the plate, which can need some hours, e.g., 6-48 hours, typically, 12-24 hours or up to 24 hours after the culture is started, i.e., after the cells have been seeded into the plate. Adherence can be visually determined. The potential therapeutic agent can also be added directly after seeding of the cells, e.g., if the agent affects adherence characteristics of the tumor cells.

Additionally, the method of the invention may comprise, after a) and b), analysing the phenotype of cells of complete dissociated tumor tissue not used for the complete dissociated tumor tissue culture of step c). The method of the invention may further comprise after step c) analysing the phenotype of cells. The analysis of the phenotype of the complete dissociated tumor tissue is preferably carried out at both time points, i.e., both before and after complete dissociated tumor tissue culture. This allows for a comparison of the phenotype and possible effects of the potential therapeutic agent. Alternatively, or preferably, additionally, the proliferation of the TIL is analysed, e.g., by flow cytometry.

This phenotypic analysis typically comprises flow cytometric analysis of TIL and tumor cells, cytokine expression and/or functional characterization of TIL. Flow cytometric analysis can comprise e.g., intracellular cytokine staining, analysis of activation, polarisation or inhibitory markers and/or other cytotoxic mediators. Analysis of expression of cytokines comprises analysis of cytokine production and/or release on the protein level (e.g., by ELISA) or analysis of cytokines expression on the mRNA level. As the inventors have found that one main factor influencing the functionality of TIL is the content of regulatory T cells, and another is the absolute number of T cells, it is preferred that the analysis of the phenotype of the TIL comprises an analysis of the content of regulatory T cells and/or of cytotoxic T cells, preferably, at least of the content of regulatory T cells. Characteristic of tumor cells, e.g., expression of immunomodulatory molecules such as TGF-β can also be determined. The assay can further determine the composition of the TME and the influence of the TME. A difference in tumor cell lysis is observed in experiments using the complete dissociated tumor tissue (with TME) compared to separated tumor cells and TIL in the absence of TME

The complete dissociated tumor tissue culture can be carried out for at least 1 day, e.g., for up to 14 days, preferably, for about 2-10 days, optionally about 5-7 days. Preferably, the efficacy of the potential therapeutic agent is analysed over that time span. The inventors have found that in this time period, effects of potential therapeutic agents on cytotoxicity can typically be optimally monitored. This time period is still significantly shorted compared to state of the art mouse models or organoids that can partially reflect effects of the immunomodulatory tumor microenvironment on functionality, in particular, cytotoxic effector functions of lymphocytes. Such models, as mentioned above, require, e.g., laboriously establishing a patient-derived tumor in a mouse or establishing an organoid from a tumor sample. While the exTuTiMo model of the inventors delivers data after 7 to 14 days, humanized mouse models and organoid or spheroid models need several months. Tissue slices can be analysed in a shorter time, but only for immune phenotyping and localization of TIL and not for their functional capacity.

The present invention also provides a method for testing efficacy of a therapeutic agent suitable for treatment of a patient having a solid cancer, comprising carrying out the method of the invention. A therapeutic agent is effective for the treatment if it improves cytotoxicity of the TIL or the other immune cells. Optionally several therapeutic agents can be compared. In parallel, an effective agent can be selected for treatment of the patient. If desired, the therapeutic agent leading to the highest improvement can be selected. Optionally, the patient can be treated with this agent.

In other words, the invention provides use of the method of the invention for testing efficacy of a therapeutic agent suitable for treatment of a patient having a solid cancer.

In a further embodiment, the invention provides a method for treating a patient having a solid cancer, comprising carrying out the method for testing efficacy of a therapeutic agent suitable for treatment of a patient having a solid cancer, and administering to the patient an effective amount of the therapeutic agent found to be effective. A combination of agents found to be effective can also be administered. For example, a combination of a TCE, e.g., a bsTCE and a cytokine such as IL-2 can be administered. IL-2 can be low-dose IL-2, i.e., administered in a dose of 50 IU/ mL. Optionally, the agent is for administration in combination with administration of ex vivo expanded TIL derived from the patient.

In another embodiment, the invention provides a complete dissociated tumor tissue culture comprising complete dissociated tumor tissue from a patient having a solid tumor, preferably, a human patient, obtainable by steps of
a) dissociating a sample derived from the tumor of the patient to produce cells of the tumor sample;
b) separating dead cells and debris to purify complete dissociated tumor tissue;
c) cultivating, in absence of a potential therapeutic agent or in presence of at least one potential therapeutic agent, the complete dissociated tumor tissue without changing the ratio between different subsets of living cells, thereby producing a complete dissociated tumor tissue culture.

Advantageously, the complete dissociated tumor tissue cells used for step c) are viable, resting and not embedded in a matrix gel. The complete dissociated tumor tissue culture can be contained in a microtiter plate, e.g., a 96 well plate, comprising a biosensor embedded at the bottom of each well, configured for determining impedance of the cell culture of the well, e.g., gold sensors. The plate can be suitable for determining impedance in a RTCA system (Agilent). It can be an E-plate. A plate as described comprising the complete dissociated tumor tissue culture of the invention is also provided.

The invention also provides use of a microtiter plate comprising a gold biosensor embedded at the bottom of each well, configured for determining impedance of the cell culture of the well, for determining the effect of a potential therapeutic agent on cytotoxicity of TIL of a patient, wherein a sample derived from a tumor of the patient is dissociated, complete dissociated tumor tissue is cultivated, in presence or absence of the potential therapeutic agent, without changing the ratio between different types of living cells, and lysis of the cells in the complete dissociated tumor tissue culture are determined, wherein the lysis of the cells is a measure of the cytotoxic effect of the TIL affected by the potential therapeutic agent. The microtiter plate is preferably used in the method of the invention. Advantageously, the cells of the complete dissociated tumor tissue that are cultured in presence or absence of the potential therapeutic agent are viable, resting and not embedded in a matrix gel.

### Fig. legends

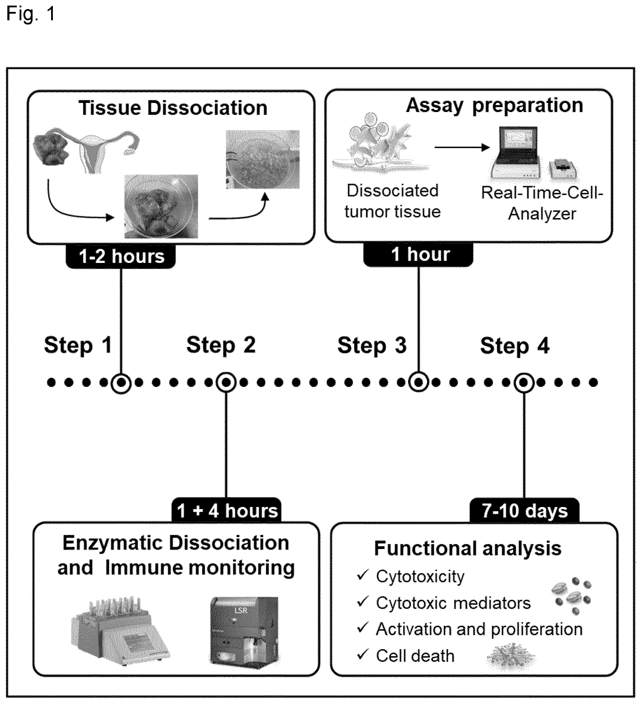

Fig. 1 Schematic overview of the method of the invention.

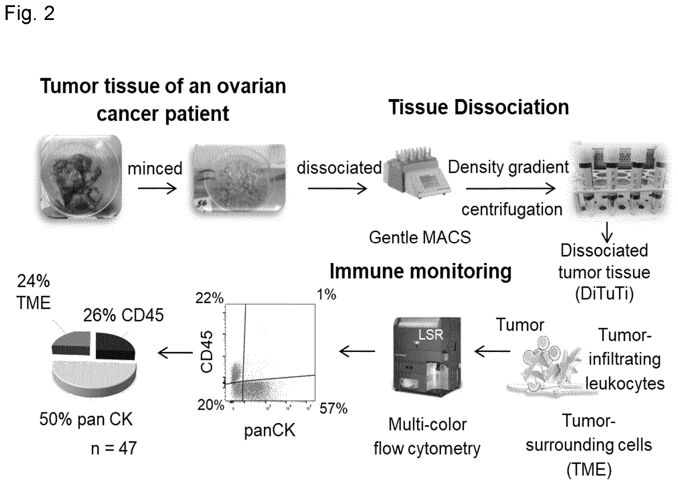

Fig. 2 Tissue Dissociation. Tumor tissues (1-100 cm³) of advanced ovarian cancer patients are washed, minced and dissociated by Gentle MACS^{®}. Possible dead cell and debris are eliminated by Pancoll^{®}-density gradient centrifugation. In the performed procedure, the composition of the dissociated tumor tissue is preserved and not manipulated. For immune monitoring, the dissociated tumor tissue (DiTuTi) is stained and analyzed by multi-color flow cytometry, to determine the percentage of the cell composition of pan Cytokeratin (panCK)-positive tumor cells, CD45-positive TIL and CD45/panCK-negative tumor stroma (tumor microenvironment, TME). Tumor tissues of 47 advanced ovarian cancer patients were analyzed.

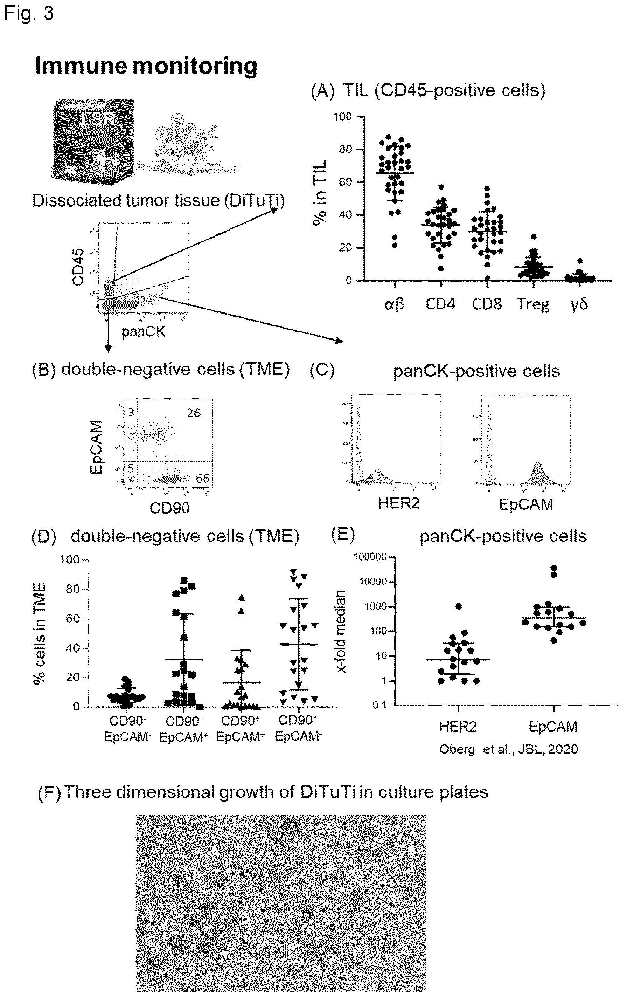

Fig. 3 Immune monitoring of tumor cells, TIL and TME from tumor tissue. Dot plot is showing distribution of panCytokeratin (panCK)-positive tumor cells, CD45⁺ leukocytes (eg, TIL) and CD45/panCK-double negative cells of the tumor microenvironment (TME) within the dissociated tumor tissue (DiTuTi). (A) The relative percentage of the different T cell subsets cells within TIL of ex vivo isolated cells of ovarian cancer patients (n = 31) were stained and analyzed by LSR-Fortessa. A gate was set on lymphocytes based on the side scatter properties and CD45-positive leukocytes to analyze γδ T cells within leukocytes. To distinguish between CD4 and CD8 αβ T cells and CD25-positive and CD127-negative regulatory T cells (Treg), a gate was set on CD3-positive cells excluding CD3/pan γδ TCR-positive cells. Each symbol presents the data of one donor, and the lines represent the median value of different independent experiments. CD45⁺ myeloid cells can also be determined but are not shown. (B) The distribution of CD90 and Epithelial cell adhesion molecule (EpCAM) on the stromal and epithelial compartment of the TME is determined within the CD45/panCK-double negative cells (TME), immediately after ex vivo isolation of the DiTuTi. (C) Histograms are showing Human epidermal growth factor receptor (HER)-2 or EpCAM expression (dark grey) compared to isotype control (light gray) on panCK-positive tumor cells also immediately after ex vivo isolation of the DiTuTi. (D/E) Flow cytometric analysis of (D) CD90 and EpCAM expression on TME and (E) HER-2 and EpCAM-positive tumor cells from 17 to 20 ovarian cancer patients are shown directly after ex vivo isolation of the DiTuTi. (D) The percentage of CD90/EpCAM expressing cells within CD45-negative TME is presented. (E) The median fluorescence intensity was calculated as an x-fold increase in relation to the staining with isotype control. Because these values do not follow a standard deviation the quartiles for each dot plot have been added. (F) A photograph of DiTuTi with the bright field setting of a microscope (EVOS M7000) demonstrates that some of the cells are forming a 3D-structure in the 96 well-plates.

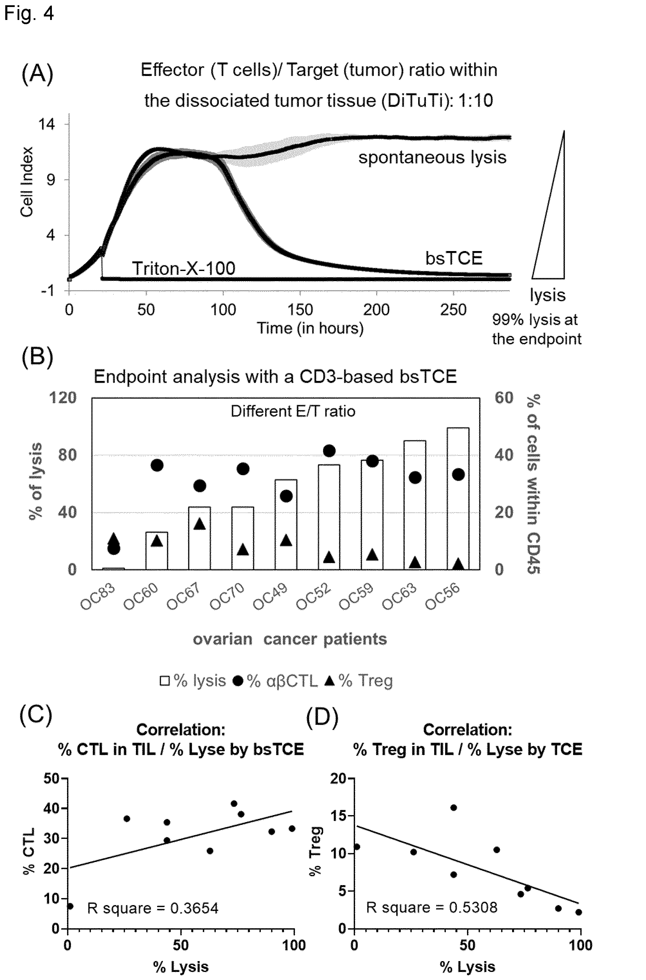

Fig. 4 Determination of CD3 T-cell cytotoxicity within dissociated tumor tissue (DiTuTi). (A) Effective lysis of tumor cells within DiTuTi. A total of 250 × 10³ ovarian DiTuTi per well were cultured in culture medium overnight. Impedance of the adherent tumor cells expressed as cell index was analyzed in 5 min steps over 25h. After reaching the linear growth phase (25h), further culture medium (black-light grey line, negative control), 1 µg/mL bsTCE [HER2xCD3] (black-dark grey line) or Triton-X-100 (black line, positive control) was added. As shown for one donor, the loss of tumor cell impedance and thus a decrease of cell index correlated with lysis of tumor cells. Lysis of tumor cells was measured in 10 min steps for additional 225h as indicated and compared to maximal lysis by Triton-X-100. The average of three replicates with SD was calculated. (B) Variable lysis of tumor cell within DiTuTi. Several further assays using DiTuTi of different donors (n = 9) in independent experiments were performed. The functional endpoint analysis indicating the lysis mediated by bsTCE targeting CD8-positive cytotoxic T cells (CTL) to tumor cells, the percentage of CTL and regulatory T cells (Treg) within DiTuTi is shown. The E [effector (T) cells]/ T (target, tumor cells) ratio differs between 10:1 and 1:90. In 5 out of 9 analyzed patients, lysis was above 60%. (C/D) Correlation data were generated with GraphPad Prism. A high number of CTL tends to improve lysis, while a high number of Treg impaired lysis. The influence of the TME is not shown in this figure.

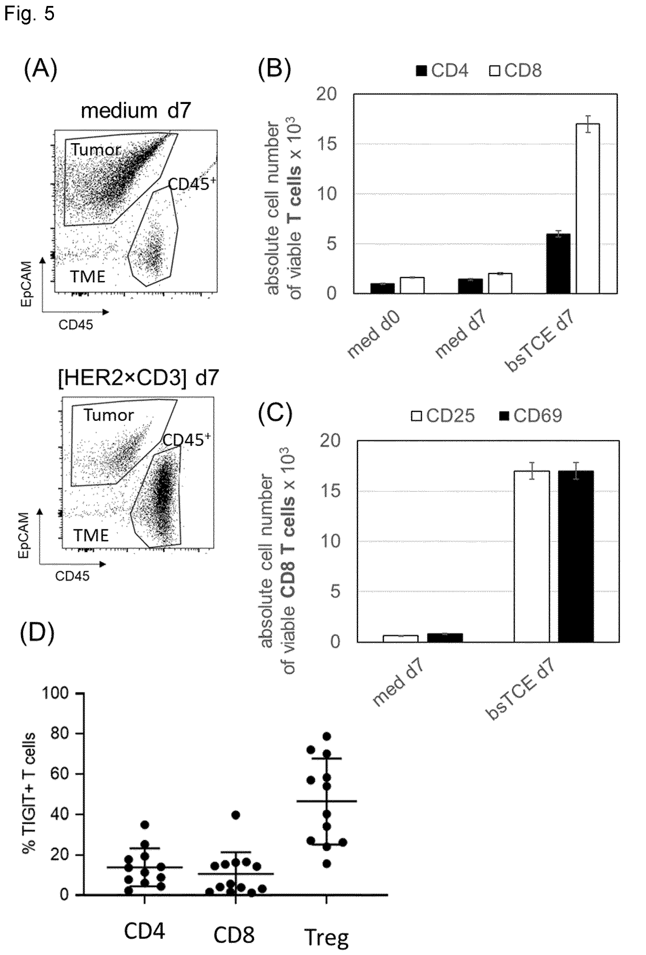

Fig. 5 Functional endpoint analysis. (A-D) Tumor cells, TIL and TME were analyzed in an endpoint analysis after culturing DiTuTi in medium (spontaneous lysis) or after stimulation by 1 µg/mL bsTCE [HER2xCD3] for 7-10 days. (A) Cell death of EpCAM-positive tumor cells mediated by bsTCE. Dot plots are showing EpCAM-positive tumor cells in the medium and their reduction (cell death) after stimulation by bsTCE, whereas the percentage of CD45-positive cells is increased and the TME decreased in the bsTCE-stimulated wells. (B) Proliferation of CD8 TIL by bsTCE. The absolute cell number of CD8 TIL is enhanced compared to CD4 TIL 7 days after stimulation with bsTCE. (C) Activation markers expressed by CD8 TIL. CD8 TIL highly expressed activation markers such as CD25 and CD69. (D) Enhanced expression of inhibitory receptor TIGIT on Treg. The inhibitory receptor T cell immunoglobulin and ITIM domain (TIGIT) is strongly expressed on regulatory T cells (Treg) compared to CD4 and CD8 T cells.

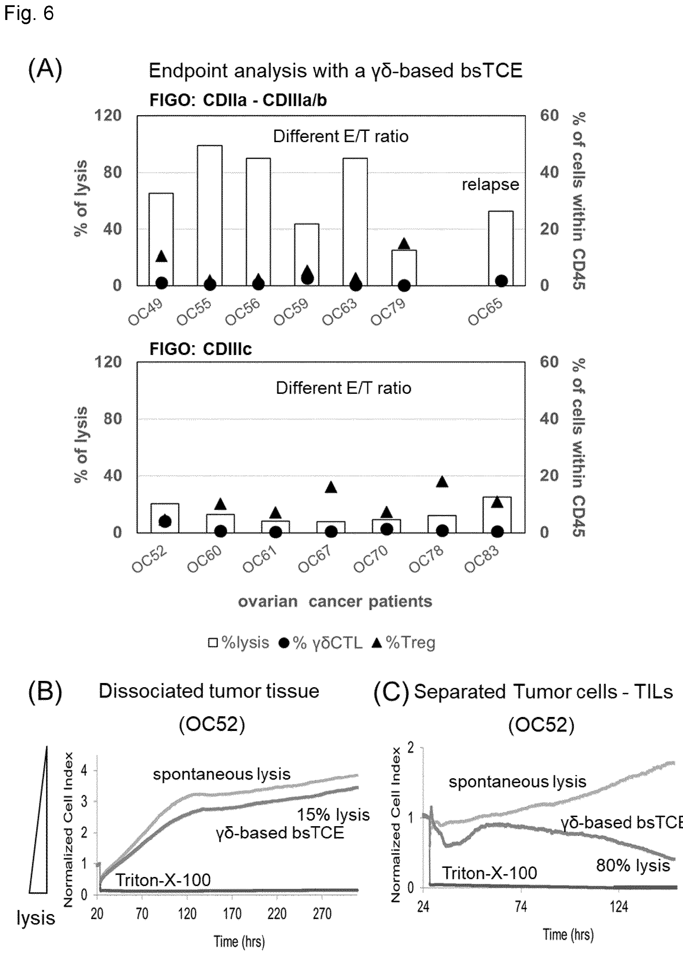

Fig. 6 Determination of γδ T-cell cytotoxicity within dissociated tumor tissue (DiTuTi) in comparison to separated cells. (A) Variable lysis of tumor cells within DiTuTi depends on the FIGO stage. Assays using DiTuTi of different donors (n = 14) in independent experiments were performed. The functional endpoint analysis indicating the lysis mediated by the γδ-based bsTCE [(HER2)₂×Vγ9] targeting Vγ9Vδ2-positive cytotoxic T cells (CTL) to tumor cells, the percentage of Vγ9Vδ2 CTL and regulatory T cells (Treg) within DiTuTi is shown. In 7 out of 14 analyzed patients in an early stage of disease (FIGO: CDlla -CDllla/b, upper panel) or with a relapse the lysis was higher compared to the other 7 donors in a more advanced stage of disease (FIGO: CDlllc, lower panel). (B/C) Comparison of DiTuTi with separated cells. (B) A total of 250 × 10³ ovarian DiTuTi per well or (C) separated tumor cells together with TIL in a comparable ratio as DuTuTi were cultured in culture medium overnight. Impedance of the adherent tumor cells expressed as cell index was analyzed in 5 min steps over 20 to 24h. After reaching the linear growth phase (20-24h), further culture medium (light grey line, negative control, spontaneous lysis), a γδ-based bsTCE ([(HER2)₂×Vγ9], dark grey line) or Triton-X-100 (black line, positive control) was added. As shown for one donor (OC52), the loss of tumor cell impedance and thus a decrease of cell index correlated with lysis of tumor cells. Lysis of tumor cells was measured in 10 min steps for additional 200-300h as indicated and compared to maximal lysis by Triton-X-100. The average of three replicates with SD was calculated. The difference between (B) and (C) is the presence or absence of TME, respectively.

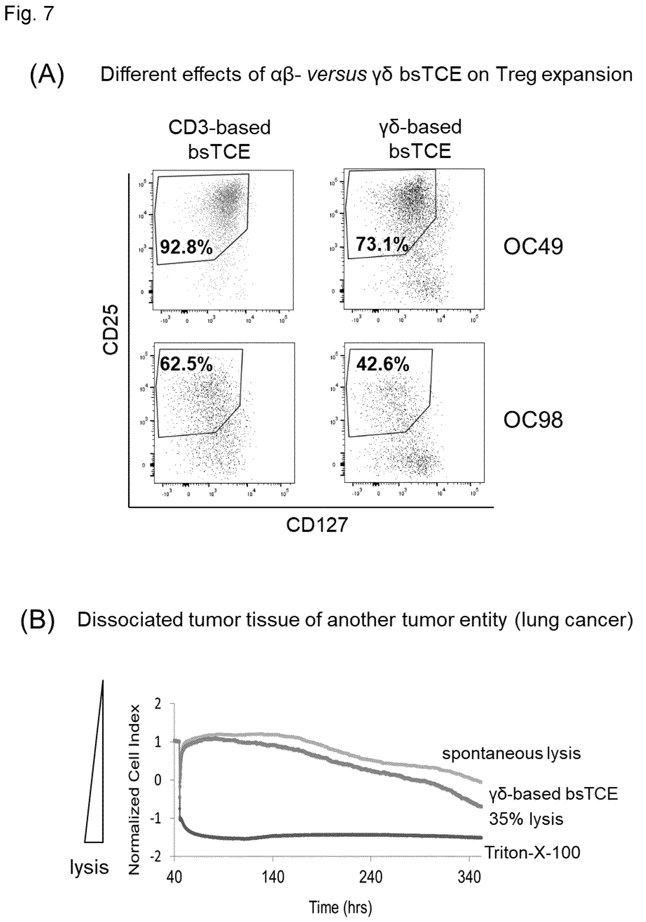

Fig. 7 Different Treg induction by CD3- *versus* γδ-based bsTCE and dissociated tumor tissue (DiTuTi) of a lung cancer patient. (A) The dot plots demonstrate the percentage of CD25-positive and CD127-negative regulatory T cells (Treg). An increase of Treg cells is shown after stimulation with CD3-based bsTCE compared to γδ-T cell-based bsTCE in both ovarian cancer patients (OC49 and OC98). (B) A total of 250 × 10³ of lung cancer DiTuTi per well was cultured in culture medium overnight. Impedance of the adherent tumor cells expressed as cell index was analyzed in 5 min steps over 40h. After reaching the linear growth phase (40h), further culture medium (light grey line, spontaneous lysis), a γδ-based bsTCE ([(HER2)₂×Vγ9], dark grey line) or Triton-X-100 (black line, positive control) was added. As shown for one donor (LC1), the loss of tumor cell impedance and thus a decrease of cell index correlated with lysis of tumor cells. Lysis of tumor cells was measured in 10 min steps for additional 340h as indicated and compared to maximal lysis by Triton-X-100. The average of three replicates with SD was calculated.

### Examples

### Material and Methods:

### Enzymatic Tissue Dissociation

A critical step to obtain freshly isolated tumor material was the dissection. On the day of the dissection, tumor tissues were washed, minced and digested. The following procedure is described for 0.5-1.5 cm³ tumor tissue.

Briefly, tumor tissue was washed in 10 cm dishes, e.g., with 10 mL PBS (pH 7.2-7.4, without Mg2⁺ or Ca2⁺) to remove blood debris. Thereafter, tumor tissue was cut into 2 to 4 mm³ pieces with a scalpel in 3 mL PBS, and the pieces were transferred into one gentleMACS^{®} C-tube (Miltenyi). The protocol should be adjusted accordingly if there are more than 1.5 cm³ tumor tissue. For instance, optimally 6 cm³ tumor tissue was cut in 12 mL PBS and distribute to four gentleMACS^{®} C-tubes. In addition, dishes were rinsed with 1.5 mL PBS and the suspension transferred to a MACS^{®} C-tube. After transfer, components A (25 µL), H (200 µL) and R (100 µL) of the Human Tumor Dissociation Kit were added following the instructions of the manufacturer (Miltenyi). MACS^{®} C-tubes were put in the gentleMACS^{®} Octo Dissociator with heater using program 37C_h_TDK_1 (Miltenyi). Thereafter, the cell suspension was centrifuged, e.g., at 481 ×g for 5 min.

After centrifugation, digested cell suspension was passed through a 100 µm cell strainer to remove cell clumping. The sample was washed twice, e.g., with 10 mL PBS at 481 ×g for 5 min. Cells were resuspend in 7 mL PBS and layed over 3 mL human Pancoll^{®} (1.077g/mL) in 10 mL polycarbonate tubes. To eliminate dead cells and debris, Pancoll^{®}-density gradient centrifugation (e.g., 751 ×g for 20 min) was performed followed by two washing steps e.g., (481 ×g for 5 min). Resuspended DiTuTi was counted and visually controlled for viability and morphology by light microscopy.

Although tumor cells and TIL differ in their size, both populations were located at the interphase after density gradient centrifugation, while dead cells and debris can be found in the pellet. Dead cells were eliminated during this step, which enhances reproducibility of the results.

### Immune monitoring

A phenotypic analysis was carried out, which helps with interpretation of the results of the cytotoxic assay. DiTuTi was stained by multicolor flow cytometry to distinguish between the different compositions such as tumor cells, tumor-surrounding cells and diverse immune subsets within CD45-positive leukocyte population. An advantage of the muti-color staining is the low number (~2 ×10⁶) of required DiTuTi.

The protocol for surface and intracellular staining or determination of absolute cell number are described under Oberg HH et al., 2020, J Leukoc Biol, 107:1081-95, doi:10.1002/JLB.5MA1119-265R, and Schadeck J/ Oberg HH et al., 2024, Front Immunol, 8:14:1286097, doi:10.3389/fimmu.2023.1286097.

All samples were analyzed on LSR-Fortessa flow cytometer (BD Biosciences) using Diva or FlowJo software when using Sytox Green solution (exclusion of dead cells) with or without BD TruCount^{™} tubes (measurement of absolute cell number > proliferation).

### Assay preparation for determination of cytotoxicity

To monitor the dynamic cellular interplay of different cells over extended time periods, the determination of immune cell cytotoxicity using the RTCA is highly recommended. The method of the invention uses the complete DiTuTi and illustrates the effects of an immunosuppressive tumor-microenvironment (TME). So far, complete DiTuTi had not been used to measure cytotoxicity of effector cells, only isolated tumor cells co-cultured with autologous effector immune cells. Therefore, no information was available about the influence of an immunosuppressive tumor microenvironment on the cytotoxic capacity of human TIL to kill autologous tumor cells. Thus, the new protocol serves as a possibility for preclinical in vitro testing of cancer drugs on freshly isolated human tumor material including immunosuppressive TME.

The present inventors found that the cell number of DiTuTi is a critical step. After extensive titration, 100.000 to 400.000 DiTuTi /well per 96 well in an E-plate is recommended, preferably, 200.000 to 250.000 DiTuTi / 96-well E-plate.

First, 50 µL of culture medium were added to a 96-well E-plate (Agilent). The E-plate was placed into the RTCA station located in a humidified 5% CO₂-incubator and background impedance measured for 15-30 min. After the background has balanced, the E-plate was removed from the RTCA station and cooled down to room temperature. 200.000 to 250.000 DiTuTi in 50 µL culture medium were added in triplicates into the 96-well E-plate. Tumor cells within the DiTuTi passively adhere at the bottom of the E-plate covered with gold electrodes. After 30 min at room temperature, the E-plate was placed into the RTCA station. The impedance of the cells expressed as an arbitrary unit called cell index (CI) was measured every 10 min for up to 24 h. After 24 h; tumor cells have reached the linear growth phase. The E-plate was taken out of the incubator and placed on pre-warmed pads inside a laminar flow to avoid a sudden drop of temperature, and the prewarmed culture medium and stimuli (i.e., potential therapeutic agents) were added. 100 µL of prewarmed culture medium (negative control) or 100 µL of stimuli such as T Cell Engager or 100 µL of 2% Triton-X-100 (positive control, 1% final concentration) were added. Cl was measured every 5 min for up to 7 to 14 days. A loss of cell index correlates with lysis.

### Functional analysis

After measuring immune cell cytotoxicity over time periods of, e.g., 7 to 14 days, a functional endpoint-analysis of immune cells was performed. This allows for the determination of expression of cytokines and alterations in expression of differentiation markers, activation and check point inhibitory molecules as well as proliferation of immune cells and cell death or regeneration of tumor cells.

The E-plate was taken out of the incubator. 100-125 µL supernatant was transferred from the E-plate to a 96-V-bottom plate. Supernatant can be stored at -20°C or directly used in an ELISA or one of the Multiplex assays to determine cytokines, chemokines and/ or cytotoxic mediators. The residual volume of the E-plate was transferred to a second 96-V-bottom plate. 100 µL PBS were added to the E-plate, and after resuspension, residual cells were transferred to the 96-V-bottom plate. 50-100 µL Trypsin/EDTA (0.05%/ 0.02%, w/v) were added to each well for 15 min. at the incubator. After 15 min., the detachment of the cells was checked with a microscope, cells were resuspended and transferred on the cooled second 96-V-bottom plate. Cells were centrifuged, e.g., at 188 ×g for 4 min. and washed, e.g., with 150 µL washing buffer at 188 ×g for 4 min. Cells were stained and incubated, e.g., for 20 min at 4°C. The cells were washed, e.g., with 150 µL washing buffer at 188 ×g for 4 min, then resuspended in 100 µL Sytox Green solution (Thermo Scientific) to measure absolute cell number of viable tumor cells and viable immune cells (cell death and proliferation).

All samples were analyzed on e.g. LSR-Fortessa flow cytometer (BD Biosciences) using Diva or FlowJo software.

### Quantification and statistical analysis

For the precise analysis of cytotoxicity, the cells were monitored every 5-10 minutes for the indicated time points. By using the RTCA software (version 2.0, Agilent) the raw data files were exported to Microsoft Excel for further calculation. The mean of Triton-X-100 samples was calculated and defined as 100% lysis at the endpoint analysis. The ratio of each sample to spontaneous lysis of DiTuTi alone was calculated and the ratio is normalized to maximal inducible lysis by Triton-X-100.

The absolute cell number of immune cells was determined, e.g., by transfer of stained cells into BD Trucount^{™} Tubes. These tubes contain a freeze-dried pellet of highly fluorescent beads with an exact number reported by the manufacturer for each charge of tubes. On average, one tube contains 5 × 10⁴ beads. The absolute cell number can be calculated following the manufacturer's instructions.

Flow cytometry analysis was performed on a LSR Fortessa running the BD Diva 9 Software and analyzed with FlowJo 10.7. Creation of figures and statistical analysis were performed with Graph Pad Prism 8 or Microsoft Excel.

### Results & Discussion:

Tumor tissue from patients were obtained from the Department of Gynecology and Obstetrics of the University Hospital Schleswig-Holstein (UKSH) in Kiel, Germany. Informed consent was obtained from all donors in accordance with the Declaration of Helsinki, and the research was approved by the relevant institutional review boards (ethic committee of the Medical faculty of the CAU to Kiel, code number: D 445/18).

In brief, tumor tissues (1-100 cm³) of advanced ovarian cancer patients were dissected in tumor tissues by the pathologists of the UKSH, Campus Kiel. Tumor tissues were washed to remove blood debris. Subsequently, the tumor tissues were minced and digested. Dead cells and debris were eliminated by density gradient centrifugation, e.g. with Pancoll^{®} (Fig. 1 and 2). The quality of tumor material can influence the dissociation to a single cell suspension and could lead to a reduced yield of cells. A fast and cooled transport of resected tumor material from the hospital via the pathology to the laboratory should be guaranteed.

The amount of tumor tissue could be a limitation. A yield between 8 and 16×10⁶ cells/g tumor tissue is expected which depends on the quality of the tumor tissue and the performance of the dissociation. Approximately 0.5 to 1 g tumor tissue (8×10⁶ cells of dissociated tumor tissue, DiTuTi) is necessary to perform a minimal approach. 2 to 4 g tissue (24×10⁶ cells of DiTuTi) is required for a complete 96-well E-plate.

In the RTCA, triplicates for each condition are recommended as well as positive and negative controls (maximal and spontaneous lysis). Depending on the RTCA system, the user is limited to one 96-well E-plate. For a higher throughput, RTCA-systems analyzing in parallel 5 or 6 96-well E-plates are available. No alternative company offers a comparable system. This approach is suitable for solid tumor entities with adherent tumor cells and can be used to a limited extent for hematological tumors with non-adherent cells.

In the performed procedure, the composition of the living cells was preserved and not manipulated. Cytotoxicity, based on monitoring cell death of tumor cells was analyzed by RTCA in the presence and absence of therapeutic agents over the course of several days.

Additionally, immune monitoring allowed the ex vivo determination of cell composition within the dissociated tumor tissue as well as the composition at the end of the cytotoxicity assay. Proliferation, cytokine release and expression of differentiation markers and activating and inhibitory molecules of immune cells were tested. Very importantly the assay depicts the influence of an immunosuppressive TME. So far, complete dissociated tumor material had not been used to determine the cytotoxic activity of TIL. Therefore, limited information exists about the influence of the TME on the cytotoxicity of TIL. Recently published studies revealed that the greatest challenge in achieving the effectiveness of cancer therapy lies in the heterogeneity of the TME, which reduces the number of TIL or their exhaustion.

Our immune monitoring data clearly demonstrated that CD4- and CD8-positive αβ T cells as well as γδ T cells and regulatory T cells infiltrate in the ovarian tumor tissue (Fig. 3). The TME comprises CD90-expressing stromal compartment and EpCAM-expressing epithelial cell as well as double-positive cells (Fig. 3). The majority of the ovarian tumor cells expressed the tumor-associated antigens HER-2 and EpCAM, which was important for the exemplary use of a bispecific antibody in our assay (Fig. 3 and 4 and Oberg et al., 2019, J Leukoc Biol 107:1081-95; doi: 10.1002/JLB.5MA1119-265R). [HER2×CD3] was used in our assay, which targeted all CD3 T cells to HER-2-expressing ovarian tumor cells (Fig .4). Alternatively, the γδ-based bsTCE [(HER2)₂×Vγ9] was used to target all Vγ9-expressing γδ T cells to HER-2 expressing ovarian or lung tumor cells (Fig. 6 and Fig. 7B and Oberg et al., 2024, Cancer Res. 74:1349-60; doi: 10.1158/0008-5472). [HER2×CD3] and [(HER2)₂×Vγ9] are just examples; other cancer drugs, e.g., other bsTCE, could also be used. The results demonstrated that the lysis of tumor cells was higher than 60% in 5 of 9 analyzed ovarian cancer patients determined after one week of culture (Fig. 4 A, B). Tumor lysis correlated with number of CD8-positive TIL and with regulatory Treg. A high number of CD8-positive TIL and a low number of Treg was beneficial for the tumor cell lysis, whereas a low number of CD8-positive TIL and a high Treg reduced the lysis (Fig. 4B/C; OC60, 67, 70) or prevent the lysis (Fig. 4B/C; OC83). Interestingly, in the latter example (OC83), in which αβ T cells failed, remaining γδ T cells partially take over the killing (Fig. 6A). Although the frequency of γδ T cells is quite low at the tumor site (<5% of the lymphocytes), they efficiently can lyse ovarian tumor cells at physiological low effector/target (E/T) ratios (1:2000 and 1:5000), when activating them by bsTCE [(HER2)₂×Vγ9] (Fig. 6A/B). Tumor cell lysis mediated by [(HER2)₂×Vγ9] rather correlated with the stage of disease and the TME than with Treg. Tumor cells of patients in an early stage of disease (FIGO: CDlla - CDllla/b) or with a relapse are lysed better compared to patients in an advanced stage of disease (FIGO: CDlllc) (Fig 6A). Additionally, a comparison of DuTuTi, which comprise TME, with separated tumor cells cocultured with autologous freshly isolated TIL in the absence of TME, demonstrated that the absence of TME has a benefit on the tumor cell lysis of patient OC52 (Fig. 6B). As shown in Fig. 5, an activation and expansion of CD8 TIL is measured after stimulation with a CD3-based bsTCE. Unfortunately, the CD3-based bsTCE also stimulated TIGIT⁺ Treg cells, which can inhibit tumor cells lysis (Fig. 5D, Fig7A). An advantage of the use of the γδ-based bsTCE [(HER2)₂×Vγ9] is the reduced activation of Treg compared to the CD3-based bsTCE [HER2×CD3] (Fig 7A).

In contrast to a humanized mouse model or organoids or spheroids, the benefit of the exTuTiMo is the evaluation of T-cell cytotoxicity under physiological E/T ratio in a human autologous model including the complete human TME. For humanized mouse models, limitations of long time growth of cells and TIL-death during this time as well as low tumor diversity is described, whereas the novel invention analyzes a high tumor diversity in a short time.

Taken together, the new preclinical method allows a cost-effective, short-time and personalized measurement of the capacity of patient's immune cells to kill tumor cells, and thereby provides information about a possible cancer treatment in just a few days.

## Claims

1. A method for determining the effect of a potential therapeutic agent on cytotoxicity of tumor-infiltrating lymphocytes (TIL) or other tumor-infiltrating immune cells of a human patient having a solid tumor, comprising steps of
a) dissociating a sample derived from the tumor of the patient to produce cells of the tumor sample;
b) separating dead cells and debris to purify complete dissociated tumor tissue;
c) cultivating, in absence of a potential therapeutic agent or in presence of at least one potential therapeutic agent, the complete dissociated tumor tissue without changing the ratio between different subsets of cells, thereby producing a complete dissociated tumor tissue culture; and
d) determining lysis or proliferation of the cells in the complete dissociated tumor tissue culture, wherein the lysis or proliferation of tumor cells is a measure of the cytotoxic effect of the TIL or the other immune cells,
wherein preferably, the cytotoxicity of TIL is determined.

2. The method of claim 1, wherein the tumor-infiltrating lymphocytes (TIL) or other tumor-infiltrating immune cells used for step c) are viable, resting and not embedded in a matrix gel.

3. The method of any of the preceding claims, wherein the effect is determined at different time points.

4. The method of any of the preceding claims, wherein the therapeutic agent is an Immune Cell Engager, preferably, a T cell engager, an immune checkpoint inhibitor, a monoclonal antibody, T cell transfer therapy and/or an immune system modulator.

5. The method of claim 4, wherein the therapeutic agent is an Immune Cell Engager, preferably, a T cell engager.

6. The method of any of the preceding claims, wherein culture of the complete dissociated tumor tissue is started the same day the tumor sample has been isolated from the patient, wherein the potential therapeutic agent is added directly or at most one day after the culture is started.

7. The method of any of the preceding claims, wherein the complete dissociated tumor tissue culture is seeded at 1×10⁵ - 4×10⁵ cells/well of a 96 well plate, preferably, in a plate comprising, in each well, an electronic sensor capable of measuring impedance.

8. The method of any of the preceding claims, wherein the lysis of adherent tumor cells is determined based on an impedance measurement, wherein, preferably, lysis of the tumor cells is determined over the course of the complete dissociated tumor tissue culture.

9. The method of any of the preceding claims, further comprising, after step a) and b), analysing the proliferation and/or phenotype of cells of complete dissociated tumor tissue not used for the complete dissociated tumor tissue culture of step c), optionally, wherein the analysis comprises flow cytometric analysis of TIL, cytokine expression and/or functional characterization of TIL.

10. The method of any of the preceding claims, further comprising, after step c) analysing the proliferation and/or phenotype of cells, optionally, wherein the analysis comprises flow cytometry analysis of TIL, cytokine expression and/or functional characterization of TIL.

11. The method of any of claims 9 and/or 10, wherein the analysis of the phenotype of the TIL comprises an analysis of the content of regulatory T cells and/or of cytotoxic T cells, preferably, at least of the content of regulatory T-cells.

12. The method of any of the preceding claims, wherein complete dissociated tumor tissue culture is carried out for at least 1-14 days, preferably, for about 2-10 days, optionally about 5-7 days,
wherein, preferably, the efficacy of the potential therapeutic agent is analysed over that time span.

13. A method for testing efficacy of a therapeutic agent suitable for treatment of a patient having a solid cancer, comprising carrying out the method of any of claims 1-12, wherein a therapeutic agent is effective for the treatment if it improves cytotoxicity of the TIL, wherein, optionally, several therapeutic agents are compared and the therapeutic agent leading to the highest improvement is selected.

14. A complete dissociated tumor tissue culture comprising complete dissociated tumor tissue from a patient having a solid tumor, preferably, a human patient, obtainable by steps of
a) dissociating a sample derived from the tumor of the patient to produce cells of the tumor sample;
b) separating dead cells and debris to purify complete dissociated tumor tissue;
c) cultivating, in absence of a potential therapeutic agent or in presence of at least one potential therapeutic agent, the complete dissociated tumor tissue without changing the ratio between different subsets of cells, thereby producing a complete dissociated tumor tissue culture,
optionally, wherein the complete dissociated tumor tissue culture is in a well-plate comprising, in each well, an electronic sensor capable of measuring impedance,
preferably, wherein the complete dissociated tumor tissue cells used for step c) are viable, resting and not embedded in a matrix gel.

15. Use of a microtiter plate comprising a gold biosensor embedded in the bottom of each well, configured for determining impedance of the cell culture of the well for determining the effect of a potential therapeutic agent on cytotoxicity of TIL or other tumor-infiltrating immune cells of a patient, comprising dissociating a sample derived from a tumor of the patient, separating dead cells and debris to purify complete dissociated tumor tissue cultivating, in absence of a potential therapeutic agent or in presence of at least one potential therapeutic agent, complete dissociated tumor tissue, wherein preferably the cells of the complete dissociated tumor tissue are viable, resting and not embedded in a matrix gel, without changing the ratio between different subsets of cells, and determining lysis of the cells in the culture, wherein the lysis of the cells is a measure of the cytotoxic effect of the TIL or the other immune cells affected by the potential therapeutic agent, using, preferably, the method of any of claims 1-13.
